Europäisches Patentamt

European Patent Office

Office européen des brevets

(11) Publication number: **0 187 422**
**A2**

# EUROPEAN PATENT APPLICATION

(21) Application number: 85202138.5

(22) Date of filing: 24.12.85

(51) Int. Cl.⁴: **C13K 1/02** , A23K 1/00 , C12P 7/10

(30) Priority: 08.01.85 GB 8500453
31.10.85 GB 8526810

(43) Date of publication of application:
16.07.86 Bulletin 86/29

(84) Designated Contracting States:
AT BE CH DE FR GB IT LI LU NL SE

(71) Applicant: SHELL INTERNATIONALE RESEARCH
MAATSCHAPPIJ B.V.
Carel van Bylandtlaan 30
NL-2596 HR Den Haag(NL)
Applicant: GIST-BROCADES N.V.
Wateringseweg 1
NL-2611 XT Delft(NL)

(72) Inventor: Harmsen, Gerrit Jan
Badhuisweg 3
NL-1031 CM Amsterdam(NL)
Inventor: Ruyter, Herman Petrus
Badhuisweg 3
NL-1031 CM Amsterdam(NL)
Inventor: French, Sidney John
30 Oaks Park Rough Common
GB-Canterbury CT2 9DP(GB)
Inventor: Van der Wal, Hans
32 St. Stephens Hill
GB-Canterbury CT2 7AX(GB)

(74) Representative: Hunter, Keith Roger Ian et al
4 York Road
London SE1 7NA(GB)

(54) Process for the treatment of biomass with steam, product thereby obtained and its use and reactor.

(57) Process for the treatment of biomass with steam in a sealed reactor at elevated temperature and pressure in a saturated steam environment comprising placing biomass in the reactor, sealing the reactor and heating the biomass until a temperature is reached in the range of 200-250°C, keeping the biomass at that temperature for 2-20 minutes before allowing it to cool gradually to room temperature while maintaining a saturated steam environment, followed by opening the reactor and recovering the steam treated biomass; biomass obtained with the process; the use in the above process of a solution containing one or more metal salts in which the biomass is soaked before it undergoes the steam treatment; process for the conversion of the treated biomass into ethanol; ethanol obtained with the latter process; animal feed containing steam treated biomass and reactor for carrying out the process.

# PROCESS FOR THE TREATMENT OF BIOMASS WITH STEAM, PRODUCT THEREBY OBTAINED AND ITS USE AND REACTOR

The present invention relates to a process for the treatment of biomass with steam.

Comminuted biomass which comprises as main components cellulose, hemicellulose and lignin and which may be derived from comminuted trees (hard wood and soft wood), plants, grasses and waste materials, such as wood chips, sawdust, chopped straw and bagasse, municipal waste and the like has long been recognised as a source of useful carbohydrates such as cellulose and sugars.

A great deal of research effort is currently being spent in this field of technology with the aim of producing sugars to be used as feed for fermentation processes optinally after additional . treatment and liquid fuels like ethanol from biomass.

From Biotechnology Letters Vol. 4 No. 3 pp. 187-192 (1982) by P. Morjanoff et. al. a two stage autohydrolyis treatment of sugar cane bagasse hereinafter referred to as "Morjanoff process" is known. In the first stage most of the hemicellulose is removed from the bagasse leaving an extracted residue and in the second stage this residue is subjected to a high pressure steaming treatment in the presence of saturated steam. In this process sugar cane bagasse was used having a particle size less than 1mm.

In· the above article the enhancing effect which the two stage autohydrolysis treatment appears to have on the enzymatic digestibility of sugar cane bagasse by hydrolysing the material with Trichoderma reesei cellulase enzymes is described.

It has now been found that biomass and in particular hard wood can be very efficiently treated with saturated steam in a sealed reactor without having to remove hemicellulose first. Surprisingly it has further been found that the steam treated product is even better digestible by enzymes than a product which has been obtained from the same starting material but which has been subjected to the "Morjanoff process". It appears to be important for getting a satisfactorily digestible product that the temperature at which the biomass is treated is above about 200°C. The enzymatic digestibility of the substrate derived from biomass which has been treated at temperatures below about 200°C appears to be insuffucient. However at temperatures higher than about 250°C decarboxylation rather than hydrolysis seems to occur. Therefore the optimal temperature is within the range of 200-250°C. Also the time of treatment is an important parameter. A suitable time range for obtaining a satisfactory result appears to be 2-20min.

Thus the present invention provides a process for the treatment of biomass with steam in a sealed reactor at elevated temperature and pressure in a saturated steam environment comprising placing biomass in the reactor, sealing the reactor and heating the biomass until a temperature is reached in the range of 200-250°C, keeping the reactor at that temperature for 2-20 minutes before allowing it to cool gradually to room temperature while maintaining a saturated steam environment, followed by opening the reactor and recovering the steam treated biomass. The biomass which is put in the reactor may be moist or dry. When necessary water should be added to ensure a saturated steam environment.

Experimentally it has been found that for achieving the best results the reactor should be kept at a temperature of 210-240°C for 4-16 minutes.

The biomass which is e.g. poplar wood, eucalyptus wood, bagasse, and/or pinewood has preferably a water content of about 15-60%wt.

In order to enable the cooling of the steam treated biomass use is made of a cooling device. For reasons of practical convenience preferably a cooling plate, which is· in contact with the high temperature steam which is present around the biomass, is employed.

Further efforts to improve the present process have resulted in the finding that if the biomass is soaked with certain metal salt solutions before it undergoes the steam treatment the enzymatic digestibility of the steamed biomass is remarkably improved even though the duration of the steam treatment is considerably reduced. Therefore the use of metal salts which are able to increase the enzymatic digestibility of the steamed biomass forms a preferred embodiment of the process according to the present invention. Preferably the salt is selected from Lewis acid catalysts and in particular from the group consisting of $AlCl_3$, $Fe_2(SO_4)_3$, $FeCl_3$ and $H_2SO_4$. For the purpose of this application $H_2SO_4$ is considered to be within the scope of metal salts as claimed.

The wood biomass can conveniently be subjected to the present process in the form of discs, chips, or flakes having suitably a thickness of 4-10mm and a diameter of 20-200mm. Especially for countries with restricted facilities but with lots of wood available, the present process may provide an important means to produce carbohydrates containing biomass which forms another feature of the present invention. This biomass may be used as feedstock for cattle and other animals which are able to digest the steam treated wood or as feedstock for fermentation after optional additional enzymatic treatment.

Therefore the present invention also provides an animal feed at least partly containing biomass which has been treated with the present steam treatment process.

The present invention further provides a reactor for the present process, comprising a sealable, elongated vessel having at the lower or upper end one or more inlets for steam and at the lower end an outlet for separating the steam-treated biomass and at the upper end one or more outlets for steam and an inlet for fresh biomass and a fixed or interchangeable cooling element. The present invention will be further illustrated with reference to the Examples.

The application of a sealed reactor has been simulated by the use of sealed glass tubes containing the biomass and enough water to ensure a saturated steam environment as further explained in Example I.

Example I

In a typical experiment 0.12g milled poplar wood of 17%w moisture content together with 0.2ml water were sealed in a heavy walled glass tube. The tube was immersed in a hot oil bath to allow the wood to be steam-treated at a temperature of 220°C for 8 minutes. After cooling in air the tube was opened and the contents assayed using the cellulase enzyme assay technique to monitor glucose release.

This standard cellulase enzyme assay is carried out as follows.

The content of the glass tube is transferred to a 25ml conical flask. To this is added pH5 buffer (phosphate/citrate) and enzymes to obtain a 5ml liquid volume containing 1mg/ml Aspergillus niger (Sigma no. C-7377) and 10mg/ml Trichodermaviride (G-B, MVA 1284) enzymes solution. The assay flasks are incubated, with agitation, at 50°C. Aliquots are removed at regular time intervals and the glucose content determined with an YSI model 23AM glucose analyzer. Results are shown in Figure I, which are further discussed in Example II.

Example II (comparative experiment)

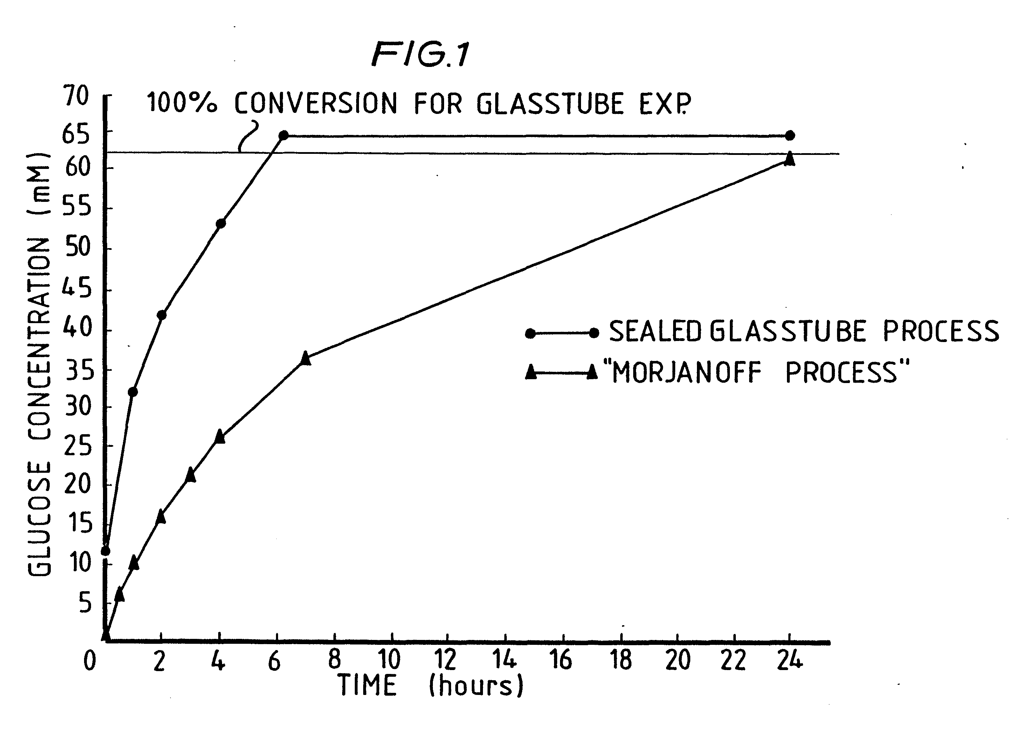

Following the method of Morjanoff as described in Biotechnology Letters Vol. 4, 3 pp. 187-192, a 6:1w/w mixture of water and poplar wood was heated in an autoclave for 1 hour at 166°C and the treated wood was then washed with water and returned to the autoclave together with sufficient water to give an 8:1w/w water-wood mixture. The autoclave was then heated up to 204°C (at an average rate of 4.6°C/min), maintained at this temperature for 5 minutes and finally allowed to cool to room temperature (an an average rate of 15°C/min). Portions of water-washed and unwashed steamed wood were then analysed by the cellulose assay and compared with a sample obtained from a glass-tube experiment as in Example I. The results are given in Fig. 1 and show a higher rate of enzymatic digestibility for the material obtained from the glass-tube experiment (total conversion after 6 hours) than for material obtained with the "Morjanoff process".

Example III (comparative experiment)

The experiment following the method of Morjanoff was repeated but now at a heating rate of 11°C/minute (which is comparable to the heating rate Morjanoff applied: 12°C/minute) and a cooling rate of 18.5°C/minute (Morjanoff:25°C/minute). Water to wood ratio in the first stage was 4.5:1 and in the second stage 7.5:1. The temperature in the second stage was 208°C for 5 minutes.

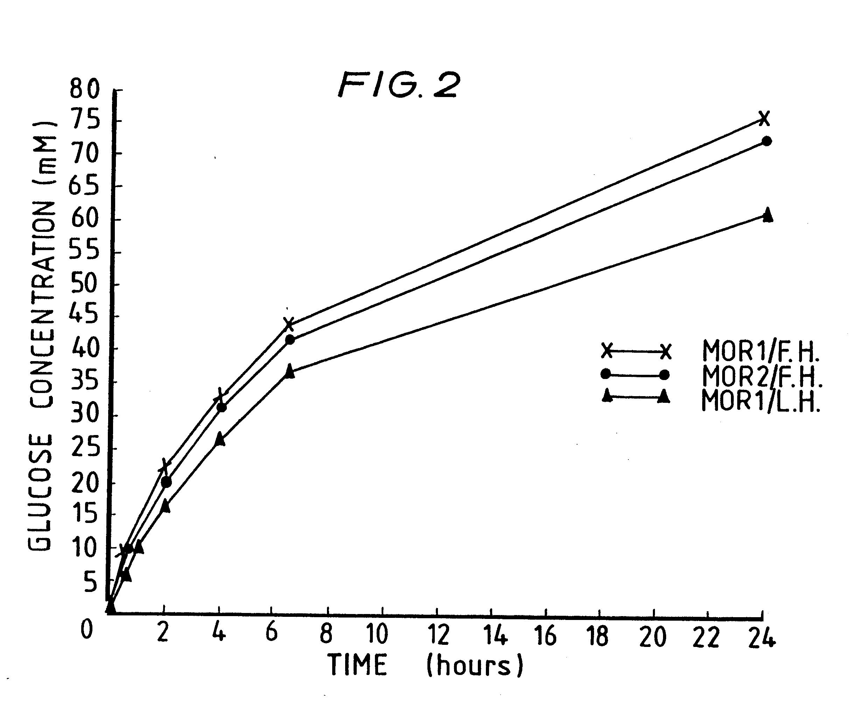

Figure 2 shows the enzymatic digestibility of two (duplo) samples thus obtained (coded MOR1/FH and MOR2/FH) * compared to that of the sample obtained by the same method but with a lower heating and a lower cooling rate (coded MOR1/LH) ** , (Example II). The digestibility of MORI/FH and MOR2/FH appears to be slightly better than MOR1/LH, but bearing in mind that for both samples the amount of substrate is a factor 1.5 higher than for sample MOR1/LH the samples MOR1/FH and MOR2/FH can be judged to be identical to the substrate of sample MOR1/LH. Thus the differences in heating rate and cooling rate between the processes as described in Examples II and III have no influence on the digestibility of the product obtained. It has thus been shown that the present steaming method produces better digestible cellulosic substrates than the Morjanoff method does.

EXAMPLE IV

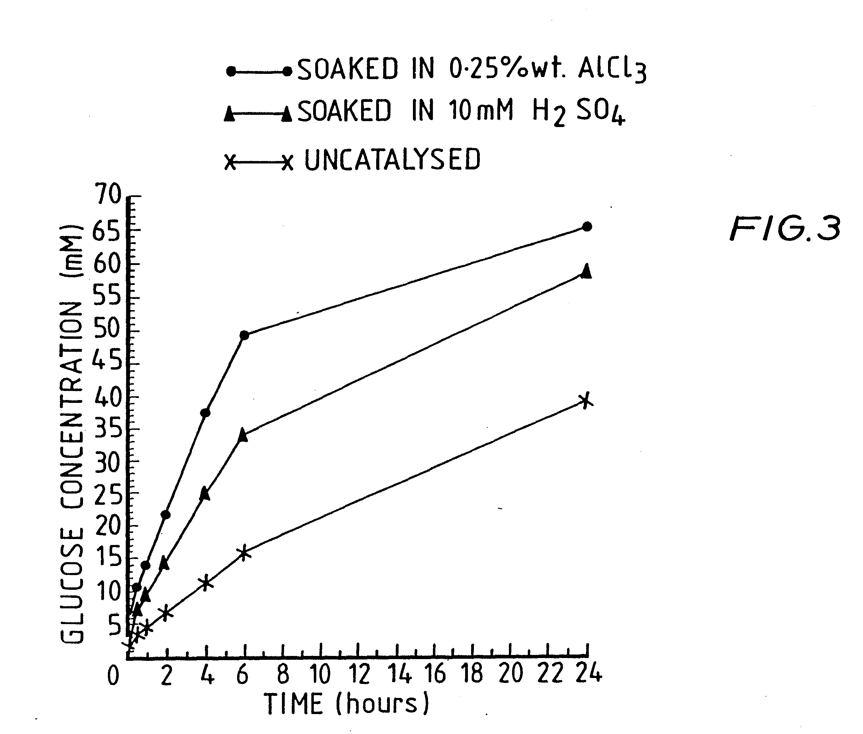

100 mg. dry wt. milled poplar wood of about 30%wt moisture was placed in a number of glasstubes. An aqueous solution of 0.25 wt $AICl_3$ or 10mM $H_2SO_4$ was added and left for 4 hours in order to leave the wood to soak. Then excess liquid was removed by centrifugation and pipetting, leaving approximately 0.4 ml of the aqueous solution in the tube. After sealing, the tubes were stored overnight and then placed in a hot oil bath for 4 minutes at 205°C. After cooling the contents were transferred to shake flasks and buffer and enzyme solutions were added as defined in Example I. The flasks were placed in a shaking waterbath (50°C) and the glucose concentration was monitored at timed intervals. From Figures 3 it can be seen that a marked improvement in enzymatic digestibility was established for the catalysed samples in comparison with the enzymatic digestibility of the uncatalysed sample.

EXAMPLE V

As a comparative experiment 100mg dry wt milled poplar wood of 30%wt moisture content was placed in a number of glasstubes after which 0.2ml 0.25%W $AICl_3$ or 10mM $H_2SO_4$ solution was added. This amount of liquid coats the wood particles rather than soaks them.

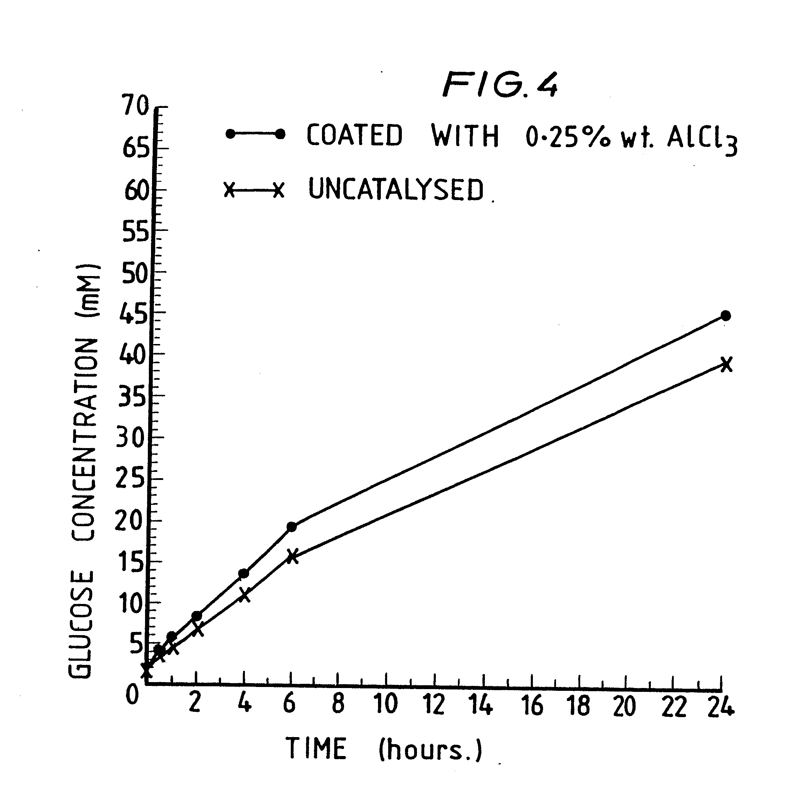

The experiment was contained as in Example IV. From Figures 4 it can be seen that although an improvement in enzymatic digestibility was established for the $AICl_3$ catalysed sample, the improvement was only modest.

Thus soaking of the wood as in Example IV is essential for obtaining a marked improvement in enzymatic digestibility as shown in Figure 3.

**Claims**

1. Process for the treatment of biomass with steam in a sealed reactor at elevated temperature and pressure in a saturated steam environment comprising placing biomass in the reactor, sealing the reactor and heating the biomass until a temperature is reached in the range of 200-250°C, keeping the biomass at that temperature for 2-20 minutes before allowing it to cool gradually to room temperature while maintaining a saturated steam environment, followed by opening the reactor and recovering the steam treated biomass.

2. Process as claimed in claim 1 in which the reactor is kept at a temperature of 210-240°C for 4-16 minutes.

3. Process as claimed in claim 1 or 2 in which the water content of the biomass is 15-60% wt.

4. Process as claimed in any one of the claims 1-3 in which for the cooling of the biomass use is made of a cooling plate in contact with the high temperature steam which is present around the biomass.

5. Process as claimed in any one of the claims 1-4 in which the cooling of the biomass is achieved by purging low temperature steam through the reactor.

6. Process as claimed in any one of the claims 1-5, in which biomass is used which has been soaked with a solution containing one or more metal salts which are able to increase the enzymatic digestibility of the steamed biomass.

7. Process as claimed in claim 6, in which the metal salt(s) is(are) selected from Lewis acid catalysts.

8. Process as claimed in claim 7, in which the Lewis acid catalyst is selected from the group consisting of $AICl_3$, $Fe_2(SO_4)_3$, $FeCl_3$ and $H_2SO_4$.

9. Process as hereinbefore described with particular reference to the Examples.

10. Biomass obtained with the process as claimed in any

---

*MOR1/FH = Morjanoff process 1st sample/Fast Heating

MOR2/FH = Morjanoff process 2nd sample/Fast Heating
**MOR1/LH = Morjanoff process 1st sample/Fast Heating one of the claims 1-9.

11. Process for the conversion of biomass as claimed in claim 10 into ethanol.

12. Ethanol obtained with a process as claimed in claim 11.

13. Animal feed at least partly containing biomass which has been treated with the process as claimed in any one of the claims 1-9.

14. A reactor for the process as claimed in any one of the claims 1-9 comprising a sealable elongated vessel having at the lower or upper end one or more inlets for steam and at the lower end an outlet for separating the steam-treated biomass and at the upper end one or more outlets for steam and an inlet for fresh biomass and a fixed or interchangeable cooling element.

FIG.1

FIG.2

Legend:
- ✕——✕ MOR1/F.H.
- ●——● MOR2/F.H.
- ▲——▲ MOR1/L.H.

FIG.3

Legend:
- ●——● SOAKED IN 0.25%wt. $AlCl_3$
- ▲——▲ SOAKED IN 10 mM $H_2SO_4$
- ✕——✕ UNCATALYSED

Y-axis: GLUCOSE CONCENTRATION (mM)

X-axis: TIME (hours)

FIG.4

● — ● COATED WITH 0·25% wt. AlCl₃

x — x UNCATALYSED

X-axis: TIME (hours.)

Y-axis: GLUCOSE CONCENTRATION (mM)

0 187 422